# EUROPÄISCHE PATENTSCHRIFT

(11) **EP 0 324 502 B1**
(45) Veröffentlichungstag und Bekanntmachung des Hinweises auf die Patenterteilung: **25.08.1993**
(21) Anmeldenummer: 89100571.2
(22) Anmeldetag: 13.01.1989
(51) Int. Cl.: A61F 13/15

(54) **Verfahren und Vorrichtung zum Befestigen eines elastischen Teils an einen tragbaren Artikel**
Method and machine for fastening a piece of elastic to an article of clothing
Procédé et dispositif pour fixer un élément élastique sur un article à porter

(30) Priorität: 14.01.1988 JP 7345/88
(43) Veröffentlichungstag der Anmeldung: 19.07.1989
(73) Patentinhaber: UNI-CHARM CORPORATION, Kawanoe-shi Ehime-ken (JP)
(72) Erfinder: Ujimoto, Hiroshi, Kawanoe-shi Ehime-ken (JP); Nomura, Hironori, Iyomishima-shi Ehime-ken (JP); Yamamoto, Toshinori, Kawanoe-shi Ehime-ken (JP)
(74) Vertreter: Sperling, Rüdiger, Dipl.-Ing.

(56) Entgegenhaltungen:
- EP-A- 119 827
- EP-A- 236 032
- US-A- 2 250 089
- US-A- 2 952 893

## Beschreibung

Die Erfindung betrifft eine Vorrichtung zum Befestigen elastischer Teile an einem tragbaren Wegwerfartikel und ein Verfahren hierzu, genauer betrifft sie gemäß Oberbegriff des Hauptanspruchs bzw. des Anspruchs 4 eine Vorrichtung und ein Verfahren zum Befestigen elastischer Teile an einer Wegwerfwindel oder Spielhose für Babys.

Eine Vorrichtung zum Befestigen elastischer Teile im Bereich der Hüftbandzone von Wegwerfartikeln und ein Verfahren, bei welchem die Vorrichtung verwendet wird, sind aus der EP 0236 032 bekannt. Bei dieser Technik muß ein elastisches Teil quer zur Laufrichtung der Bahn gestreckt und auf eine vorbestimmte Länge geschnitten werden, wenn es sich noch im gedehnten Zustand befindet.

Die dabei jeweils erhaltenen einzelnen elastischen Teile werden mittels eines Klebers so befestigt, daß sie sich in Querrichtung der kontinuierlichen Bahn erstrecken.

Bei der genannten europäischen Patentschrift wird der Dehnvorgang auf einer Trommel durchgeführt, die über ihren Umfang verteilt fünf Dehnanordnungen aufweist, welche das querliegend angelieferte abgeschnittene elastische Teil an beiden Enden gleichzeitig ergreift und während ca. einer halben Umdrehung der Trommel auf die vorbestimmte Länge dehnt und im gedehnten Zustand auf die Halbfertigbahn aufpreßt. Während der halben Umdrehung der Trommel werden die Greifklauenpaare, die jeweils ein elastisches Teil ergreifen über eine Zwangsführung um den gewünschten Abstand voneinander entfernt.

Es haben sich jedoch Schwierigkeiten bei der Handhabung der elastischen Teile bei den hohen Geschwindigkeiten ergeben, die für eine Massenfertigung erforderlich sind. Das gleiche gilt für das Befestigen der elastischen Teile an der Bahn, die ebenfalls mit hoher Geschwindigkeit zugeführt werden. Eine geeignete Lösung für ein derartiges Hochgeschwindigkeitshandling ist bisher noch nicht gefunden worden.

Die Aufgabe der Erfindung besteht darin, eine Vorrichtung der genannten Art bereitzustellen, die bei einfachem und kostengünstigem Aufbau bei sehr hohen Geschwindigkeiten, die für eine effizientere Massenproduktion erforderlich sind, zuverlässig arbeitet und leicht in eine existierende Produktionslinie eingebaut werden kann.

Des weiteren liegt der Erfindung die Aufgabe zugrunde, ein Verfahren zum wirkungsvollen Befestigen eines elastischen Teils an den Hüftbandbereich eines Artikels bei hoher Arbeitsgeschwindigkeit bereitzustellen, wobei der Artikel ebenfalls mit hoher Geschwindigkeit gehandhabt sowie an eine kontinuierliche Bahn eines Grundmaterials oder eines Halbfertigteils des Artikels angebracht wird.

Die Aufgabe wird hinsichtlich der Vorrichtung zum Befestigen eines elastischen Teils an einem tragbaren Artikel durch die Merkmale des kennzeichenden Teils des Hauptanspruchs gelöst.

Die Mittel zum Trennen der kontinuierlichen Bahn sind vorteilhafterweise so angeordnet, daß sich die Trennachse durch einen vorbestimmten Abschnitt der jeweiligen aufeinanderfolgenden Teile erstreckt, so daß jedes elastische Teil oder Element in Förderrichtung der kontinuierlichen Bahn in zwei Teile aufgetrennt wird, welche beiden Teile jeweils an sich gegenüberliegenden Enden von aufeinanderfolgenden individuellen Artikeln angeordnet sind.

Die Mittel zum Zuführen, zum Beabstanden und zum Dehnen umfassen vorzugsweise erste und zweite elastische Greifmittel für das elastische Teil; diese sind so angeordnet, daß sie jeweils einander gegenüberliegende Querenden der aufeinanderfolgenden einzelnen elastischen Teile ergreifen können. Es sind Mittel zum Bewegen der jeweiligen Greifmittel entlang divergierender Zuführungsbahnen vorgesehen, die sich von den Trennmitteln zu den Transfermitteln erstrecken und dabei progressiv die elastischen Teile während der Zuführungsbewegung in Querrichtung dehnen, um graduell die Abmessung in Querrichtung des elastischen Teils zu vergrößern. Im einzelnen umfassen die Zuführungs-, Transfer- und Dehnmittel erste und zweite Walzenpaare, erste und zweite Paare von Endlosgurten, die um die jeweiligen Walzenpaare herumlaufen und dabei angetrieben werden, wobei die Walzen jedes Walzenpaars in Zuführungsrichtung einen Abstand voneinander aufweisen und jeweils den Trennmitteln und den Transfermitteln benachbart sind.

Des weiteren sind Mittel vorgesehen zum Halten der Gurte auf den Walzen, um diese in einem divergierenden Verhältnis zueinander zu halten, wenn sie sich in Führungsrichtung von den Trennmitteln zu den Transfermitteln hin erstrecken, wobei die Gurte des ersten Gurtpaars mit dem gleichen Winkel divergieren, wie die Gurte des zweiten Gurtpaars. Die ersten und zweiten Walzen sind jeweils in einander gegenläufigem Sinn angetrieben und so angeordnet, daß das treibende Trumm der Gurte des ersten Gurtpaars, das sich in Zuführungsrrichtung erstreckt, mit dem getriebenen Trumm der jeweiligen Gurte des zweiten Gurtpaars fluchten und diesen benachbart liegen, so daß die jeweiligen Gurte des ersten Paars mit den jeweiligen Gurten des zweiten Paars zusammenarbeiten, um zwischen sich die Enden des elastischen Teils zu ergreifen und dabei sukzessive elastische Teile von der Schneidestation zur Transferstation fördern und gleichzeitig diese elastischen Teile während der Zuführbewegung progressiv in Querrichtung dehnen. Die Dehnungs- und Zuführungsvorrichtung ist eine relativ einfache Konstruktion mit einem Minimum an Vibrationen, die im wesentlichen keine reziprok arbeitenden Teile erfordert und leicht an vorhandene Artikelproduktionslinien angebaut oder integriert werden kann.

Erfindungsgemäß wird das Verfahren zum Befestigen eines elastischen Teils an einem am Körper zu tragenden Artikel unter Verwendung der Vorrichtung nach den Ansprüchen 1 - 3 durch die im Kennzeichen des Anspruchs 4 angegebenen Verfahrenschritte gelöst.

Das progressive Dehnen der einzelnen elastischen Teile auf den divergierenden Gurten, das ihre sich in Querrichtung erstreckende Länge graduell vergrößert, während sie in Richtung der Halbfertigteilbahn des Artikels gefördert wird, erleichtert ein glattes kontinuierliches und stabiles Arbeiten bei minimaler Vibration, was besonders wichtig ist für einen zuverlässigen Betrieb bei einem kontinuierlichen Hochgeschwindigkeitsprozeß.

Die Trennachse geht durch vorbestimmte Bereiche der jeweiligen elastischen Teile, um jedes elastische Teil in der Laufrichtung in zwei Teile zu teilen, wobei die getrennten Einzelteile jeweils auf den sich gegenüberliegenden Enden zweier aufeinanderfolgender Artikel angeordnet sind.

Die Verfahrensschritte des Abtrennens der Stücke entlang einer Trennlinie von der Bahn, um die individuellen Artikel ergibt eine Erleichterung der Betriebsoperation, da lediglich ein einziger Schnitt erforderlich ist, um jeweils ein Hüftband an einander gegenübeliegenden Enden aufeinanderfolgender Artikel zu schaffen und um die einzelnen Artikel von der Bahn abzutrennen.

Im folgenden wird ein Ausführungsbeispiel einer Vorrichtung zum Durchführen des erfindungsgemäßen Verfahrens anhand der begleitenden Zeichnungen näher erläutert. Es zeigen:
Fig. 1 eine Draufsicht auf einen tragbaren Artikel, hier eine Wegwerfwindel, mit teilweise weggebrochenen Bereichen, die auf der erfindungsgemäßen Vorrichtung und mit dem erfindungsgemäßen Verfahren hergestellt worden ist,
Fig. 2 eine perspektivische Ansicht auf einen Teil der Vorrichtung mit Mitteln zum Auftragen eines Klebers auf eine kontinuierliche elastische Lage und mit Mitteln zum Abtrennen der elastischen Lage, um nacheinander einzelne elastische Teile bereitzustellen,
Fig. 3 eine perspektivische Ansicht eines Teils der Vorrichtung zum Dehnen und Übertragen der einzelnen elastischen Teile,
Fig. 4 eine schematische Ansicht von oben auf einen oberen Teil des Abschnitts der Vorrichtung, der in Fig. 3 dargestellt ist,
Fig. 5 eine Seitenansicht, die schematisch einen Bereich der Vorrichtung zeigt, in den nacheinander elastische Teile auf eine kontinuierliche Bahn von Artikelhalbfertigteilen geklebt werden, welche um eine Trommel herumgeführt wird,
Fig. 6 eine Draufsicht auf die die elastischen Teile tragende Bahn, und
Fig. 7 eine Seitenansicht einer geeigneten Vorrichtung bekannten Typs zum Abtrennen der Bahn.

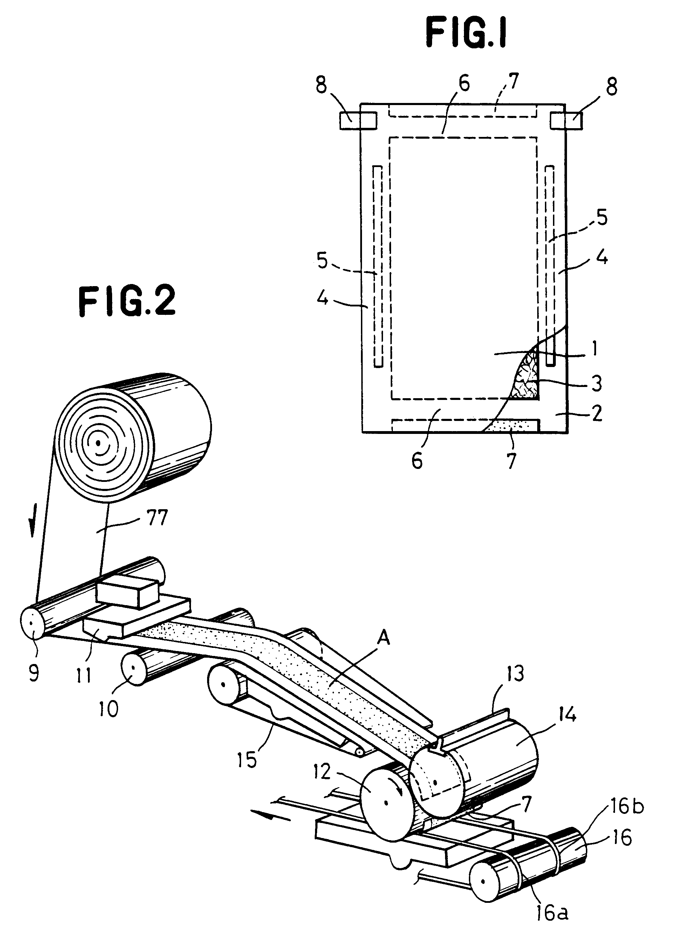

In Fig. 1 ist eine Wegwerfwindel mit einer feuchtigkeitsdurchlässigen vorderen Lage 1, einer feuchtigkeitsundurchlässigen rückseitigen Lage 2 und mit einem zwischen diesen beiden Lagen angeordneten Saugkern 3 dargestellt. An Beinseitenklappen 4, die sich in Längsrichtung der Windel erstrecken, sind elastische Teile 5 für den Beinkontakt befestigt. Die Befestigung erfolgt während sich die elastischen Teile in Längsrichtung in gedehntem Zustand befinden. Jede sich in Längsrichtung erstreckende Beinseitenklappe 4 wird durch überlappende Randabschnitte der beiden Lagen 1 und 2 definiert, welche sich seitlich über die Längsränder des Saugkerns 3 erstrecken. Hüftbänder bildende elastische Teile 7 sind in sich in Längsrichtung erstreckende Seitenklappen 6 eingeklebt, wobei das Einkleben im in Querrichtung gedehnten Zustand der elastischen Teile erfolgt. Jede Hüftseitenklappe wird durch überlappende Abschnitte der Lagen 1 und 2 gebildet, die sich in Längsrichtung über die Seitenränder des Saugkerns 3 erstrecken. An der rückseitigen Lage 2 sind an den Schnittstellen einer sich seitlich erstreckenden Endklappe und sich in Längsrichtung erstreckenden Seitenklappe Bandbefestiger 8 vorgesehen.

In Fig. 2 ist ein Teil einer Vorrichtung zum Aufbringen eines Klebstoffs auf eine kontinuierliche elastische Lage dargestellt. Eine Zuführungsrolle trägt eine kontinuierliche elastische Lage 77 vorbestimmter Breite, die vorzugsweise aus einem Polyurethanschaum besteht. Die kontinuierliche elastische Lage wird in einer Schneidestation zwischen einem Paar Schneidwalzen 12 und 14 durchgezogen, von denen die Walze 14 mit einem Messer 13 versehen ist. Eine Auftragvorrichtung für ein Klebmittel ist so angeordnet, daß sie heißes geschmolzenes Klebmittel A auf eine Oberfläche der kontinuierlichen elastischen Lage auftragen kann, wobei diejenigen Abschnitte der Oberfläche freigelassen werden, die den sich in Längsrichtung erstreckenden Randabschnitten benachbart sind. Auf der kontinuierlichen elastischen Lage sind somit die beiden Randstreifen freigelassen, während das Klebmittel in einem Mittenbereich aufgetragen ist. Die Klebmittelbeschichtung A kann in kontinuierlicher, intermittierender oder punktförmiger Weise aufgetragen sein.

Während des Durchgangs zwischen dem Schneidwalzenpaar 12 und 14 wird das vorangehende Ende der elastischen Lage 77 wiederholt durch das Messer 13 entlang einer sich in Querrichtung erstreckenden Trennlinie abgeschnitten, wodurch nacheinander einzelne elastische Teile vorbestimmter Länge gebildet werden. Die Außenumfangsfläche der Walze 14 ist mit einem Silikonharz beschichtet, um zu verhindern, daß das Klebmittel an ihr haften bleibt. Zwischen einer Walze 10 und den Schneidwalzen 12, 14 ist eine Saugvorrichtung 15 angeordnet, um ein Auswandern der elastischen Lage 17 zu verhindern und um eine ausreichende Kraft zur Verfügung zu stellen, um die elastische Lage zu halten, während sie zugeführt wird.

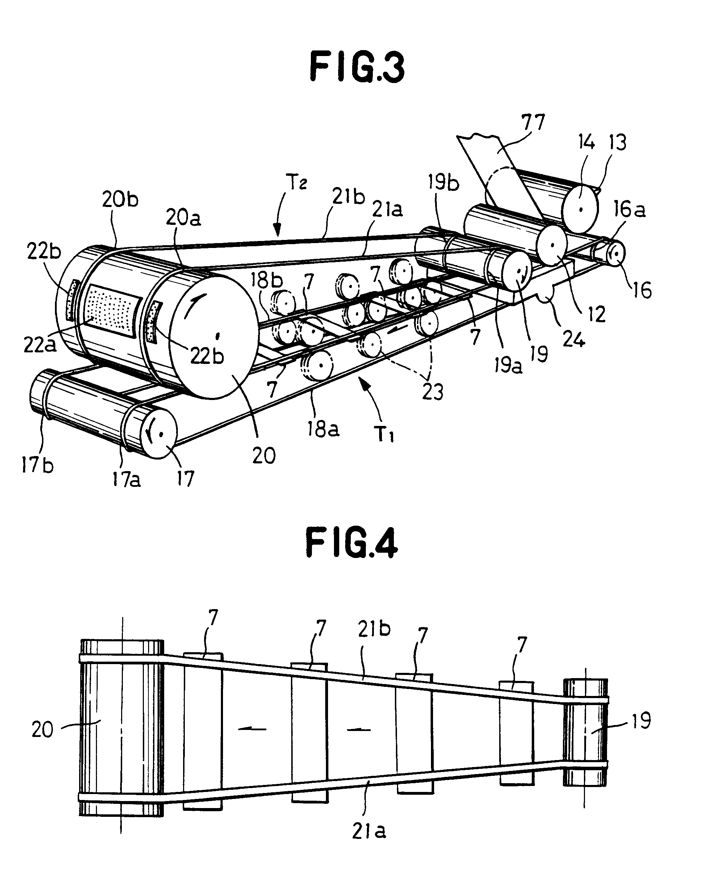

In Förderrichtung abwärts von den Walzen 12, 14 sind Dehn- und Zuführungsvorrichtungen T₁, T₂, 16 - 20b, wie in Fig. 3 dargestellt, angeordnet. Die Teil-Vorrichtung T₁ der Dehn- und Zuführungsmittel weist eine Lineargeschwindigkeit auf, die gleich ist derjenigen der Teil-Vorrichtung T₂, jedoch höher als die Umfangslineargeschwindigkeit der Walzen 12, 14. Die Vorrichtung T₁ umfaßt eine getriebene Walze 16, die dicht an den Walzen 12, 14 angeordnet ist und in Förderrichtung abwärts von der getriebenen Walze 16 eine Antriebswalze 17 sowie Gurte 18a, 18b, die um die Walzen 16 und 17 herumgelegt sind. Die Gurte 18a, 18b weisen jeweils einen V-förmigen Querschnitt auf und greifen in V-förmige Nuten 16a, 16b und 17a, 17b ein, die sich jeweils auf dem Umfang der Walzen 16 und 17 erstrecken.

Die V-förmigen Nuten der beiden Walzen 16 und 17 sind an ihren jeweiligen, sich in Umfangsrichtung erstreckenden Oberflächen an Stellen angeordnet, die ihren Seitenrändern benachbart sind, wobei der Abstand zwischen den Nuten 17a und 17b größer ist als zwischen den Nuten 16a und 16b, so daß der Abstand zwischen den Gurten 18a, 18b progressiv zunimmt, während sie sich von der Walze 16 zu der Walze 17 hin erstrecken.

Die Vorrichtung T₂ ist an einer oberen Fläche oder oberen Seite, definiert durch die Vorrichtung T₁, angeordnet und umfaßt eine getriebene Walze 19, die in Förderrichtung stromabwärts sowohl der Walze als auch der Schneidewalzen 12 und 14 angeordnet ist, sowie stromabwärts der Walze 19 und stromaufwärts der Walze 17 gelegen, eine trommelförmige Antriebswalze 20, die gleichzeitig als Transfermittel für die gedehnten elastischen Teile zu den Halbfertigteilen dient, und Gurte 21a und 21b, die um die Walzen 19 und 20 herumgeführt sind. Die Gurte 21a und 21b haben ebenfalls jeweils einen V-förmigen Querschnitt, der ähnlich ist demjenigen der Gurte und greifen in V-förmige Nuten 19A, 19B und 20a, 20b ein, die auf den jeweiligen Umfangsflächen der jeweiligen Walzen 19 und 20, den Rändern benachbart ausgebildet sind. Der Abstand zwischen den Nuten 19A, 19B ist geringer als zwischen den Nuten 20a, 20b, so daß die Gurte 21a, 21b progressiv divergieren, während sie sich von der Walze 19 zur Walze 20 hin erstrecken, wobei die relative Trennung der Nuten 19A, 19B und 20a, 20b derart gewählt ist, daß die Gurte 21a, 21b mit dem gleichen Winkel divergieren, wie die Gurte 18a, 18b, so daß sie diesen Gurten 18a, 18b über ihre gesamte Länge in Zuführrichtung benachbart sind.

In der Walze 20 ist ein Paar nicht dargestellter Saugvorrichtungen angeordnet, die aktive Saugmündungen 22a, 22b aufweisen, die an sich einander gegenüberliegenden Abschnitten der Umfangsfläche der Walze 20 ausmünden. Die Saugmündung 22a ist zwischen den Nuten 20a, 20b angeordnet, während die Saugmündung 22b an gegenüberliegenden Seiten der Saugfläche 22a außerhalb der Nuten 21a, 21b angeordnet sind. Da Trommelwalzen mit Saugvorrichtungen bekannt sind, werden nähere Einzelheiten hierzu nicht erläutert. Zwischen dem vorderen und dem rückwärtigen Abschnitt der Gurte 18a, 18b sowie benachbart und oberhalb der vorderen Abschnitte der Gurte 21a, 21b sind an vorbestimmten Stellen in ihrer Bewegungsrichtung eine Vielzahl von Führungsrollen 23 mit jeweils V-förmiger Nut vorgesehen, um einen stabilen Lauf der Gurte während ihres Eingriffs mit den elastischen Teilen sicherzustellen, während ein vorbestimmter Spalt zwischen ihnen beibehalten wird.

Unterhalb eines Bereichs, in dem die individuellen elastischen Teile 7 vom Streifenausgang getrennt und von den Schneidewalzen 12, 14 abgezogen werden, ist eine Saugvorrichtung 24 angeordnet, die eine genügend große Saugkraft bereitstellt, um ein elastisches Teil zu halten, während es durch die Gurte 18a, 18b und 21a, 21b ergriffen wird, um weitergefördert zu werden.

Die Saugvorrichtung 24 legt das elastische Teil auf einen nach vorne laufenden Abschnitt der sich bewegenden Gurte 18a, 18b; unmittelbar danach wird das elastische Teil an den Längsrändern, an denen kein Klebmittel aufgetragen worden ist, als Ergebnis der Vorwärtsbewegung der Gurte zwischen den Gurten 18a, 18b und den Gurten 21a, 21b ergriffen und weitergefördert. Zu dieser Zeit wurde das vorhergehende elastische Teil 7 von einem nachfolgenden Teil 7 in Laufrichtung durch einen vorbestimmten Betrag getrennt, da die Gurte 18a, 18b, 21a, 21b eine höhere lineare Geschwindigkeit aufweisen als die Umfangsgeschwindigkeit der Schneidewalzen 12, 14. Der Abstand des nachfolgenden elastischen Teils 7 entspricht dem erforderlichen Abstand, um eine synchrone Erfassung der nachfolgenden Teile durch die Saugmündungen 22a, 22b der rotierenden Walze 22 zu halten. Die aufeinanderfolgenden elastischen Teile werden als Ergebnis des Divergierens der Gurte in Förderrichtung zu einem bestimmten gedehnten Zustand in Querrichtung gedehnt, während sie zur Walze 20 gefördert werden. Die gedehnten elastischen Teile treffen synchron mit den Saugmündungen 22a, 22b der rotierenden Walze 22 zusammen, so daß ein Zentralabschnitt einer unbeschichteten Oberfläche den Mündungen 22a zugewandet ist und von diesen gehalten wird, während die unbeschichteten Seitenränder durch die Saugmündungen 22b gehalten werden.

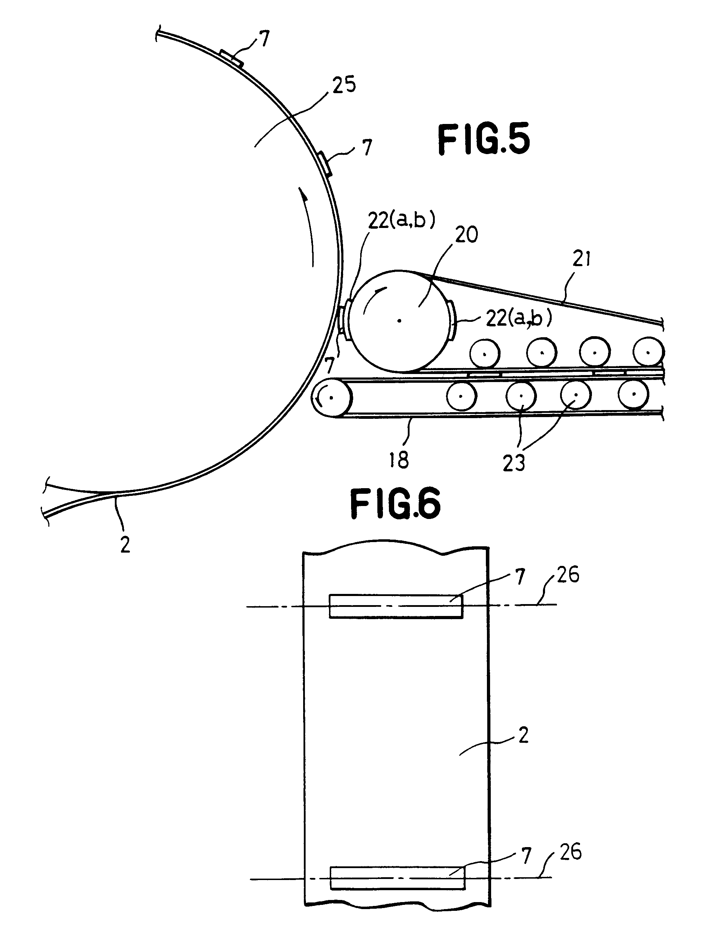

Wie aus Fig. 5 zu erkennen ist, ist eine trommelförmige Walze 25 mit einer um ihre Außenumfangsfläche herumgeführte, eine kontinuierliche Bahn definierenden rückwärtigen Lage 2 (oder das Halbfertigteil des Artikels) der Umfangsoberfläche der Walze 20 gegenüberliegend angeordnet, so daß die beschichtete Oberfläche der aufeinanderfolgenden elastischen Teile gegen die Saugmündungen 22a, 22b gehalten werden, dort anhaften und dabei auf die rückwärtige Lage übertragen werden, wobei dann augenblicklich die Saugaktion der Saugmündungen 22a, 22b, die das elastische Teil halten, unterbrochen wird. Als Ergebnis sind die elastischen Teile, wie in den Fig. 5 und 6 zu erkennen ist, an die rückseitige Lage 2 auf der Walze in vorbestimmten Abständen auf deren Umfang angeklebt, d.h. in Längsrichtung (Laufrichtung) der rückseitigen Lage, wobei sie in Querrichtung in gestrecktem Zustand darauf gehalten sind. Es wird darauf hingewiesen, daß der Abstand oder das Intervall zwischen den elastischen, zu übertragenden Teilen geändert werden kann durch Ändern der jeweiligen linearen Umfangsgeschwindigkeiten der Walze 20 und der Walze 25.

Obwohl es nicht in der Zeichnung dargestellt ist, weist die rückwärtige Lage 2 andere Artikelteile wie beispielsweise eine kontinuierliche Oberlage, den zwischen der rückseitigen Lage und der Oberlage eingesetzten Saugkern und die sich in Längsrichtung oder kontinuierlich erstreckenden elastischen Teile auf, wobei ein kontinuierlicher Streifen von individuellen Artikeln in unmittelbarer Reihenfolge erzeugt wird. Der kontinuierliche Streifen wird dann entlang einer Trennachse, die sich in Querrichtung durch das Zentrum der aufeinanderfolgenden jeweiligen elastischen Teile erstreckt, durch Betätigung der Schneidewalzen, die einen ähnlichen Aufbau wie in Fig. 2 gezeigt, aufweisen, voneinander getrennt, und in Laufrichtung abwärts auf die Walze 25 angeordnet, wobei die indi-ö viduellen Artikel von dem Streifen getrennt werden und gleichzeitig Abschnitte des zweigeteilten elastischen Teils im Hüftbandbereich durch einen einzigen Schneidschlag geschaffen werden.

Obwohl bei dieser Ausführungsform der Erfindung die elastischen Teile auf der rückseitigen Lage angebracht werden, ist es leicht ersichtlich, daß das elastische Teil stattdessen auch auf einer kontinuierlichen Bahn angebracht sein kann, die die obere oder vordere Lage bildet.

Bei dem oben beschriebenen Ausführungsbeispiel eines Verfahrens und einer Vorrichtung zum Anbringen eines elastischen Teils auf einem tragbaren Gegenstand werden individuelle elastische Teile durch wiederholtes Schneiden des Kopfendes einer kontinuierlichen elastischen Lage in Querrichtung nach dem Beschichten mit Klebstoff erhalten. Die einzelnen elastischen Teile werden in Querrichtung auf eine vorbestimmte Länge gedehntz und auf eine kontinuierliche Bahn in vorbestimmten Abständen entlang einer kontinuierlichen Bahn angebracht, die einen Teil eines Vorläufers oder Halbfertigteil eines tragbaren Artikels bildet. Auf diese Weise kann die Produktionslinie für die kontinuierliche elastische Lage und individuelle elastische Teile auf derselben Linie arrangiert werden, welche die anderen Teile, die den Artikel bilden, produzieren, wodurch eine relative Geschwindigkeitszunahme beider Produktionslinien erzielt werden kann, so daß der Gegenstand mit den elastischen Teilen, die sich im rechten Winkel zur Längsabmessung der Artikel erstrecken, mit geringeren Kosten hergestellt werden können.

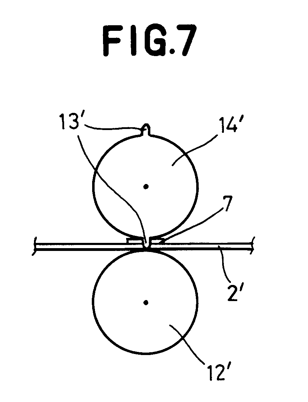

In Fig. 7 sind Mittel zum Trennen der kontinuierlichen Bahn 2 entlang der Trennlinie 26 dargestellt. Es ist lediglich ein Schnitt entlang der Trennlinie 26 notwendig, um die elastischen Teile zu trennen, um zwei Hüftbandbereiche zu bilden, und zwar an jedem jeweiligen benachbarten Ende der Artikel und um nacheinander die Artikel von der kontinuierlichen Bahn abzutrennen. Diese Schneidvorrichtung mit den Walzen 12', 14'und dem Messer 13'ist ähnlich aufgebaut wie die oben beschriebene Vorrichtung 12-14. Die erfindungsgemäße Vorrichtung weist einen relativ einfachen Aufbau auf und kann leicht zu einer den Gegenstand erzeugenden Herstellinie zusammengebaut werden.

## Patentansprüche

1. Vorrichtung zum Befestigen eines elastischen Teils an einem tragbaren Artikel, mit den folgenden Merkmalen:
- Mittel (9,10,15) zum Zuführen einer kontinuierlichen elastischen Lage (77) vorbestimmter Breite von einem Vorrat zu einer Schneidestation (12,13,14)
- Mittel (11) zum kontinuierlichen Auftragen einer Klebbeschichtung A auf eine Oberfläche der elastischen Lage mit Ausnahme der sich in Längsrichtung erstreckenden Randbereiche,
- Zuführungsmittel (24,T₁,T₂), die sich von der Schneidestation zu Transfermitteln (20) erstrecken, um die in der Schneidestation quer zur Bahn abgeschnittenen individuellen, aufeinanderfolgend gebildeten elastischen Teile (7) von der Schneidestation zu den Transfermitteln zu transportieren, währenddessen die elastischen Teile auf eine vorbestimmte Distanz in Laufrichtung voneinander beabstandet werden,
- Mittel zum Dehnen (T₁,T₂,16,17,19,20,23) der elastischen Teile in einen gestreckten Zustand in Querrichtung,
- Mittel (25) zum Zuführen einer kontinuierlichen Bahn (2) aus Artikelhalbfertigteilen zu den Transfermitteln, die so angeordnet sind, daß die beschichtete Oberfläche jedes elastischen Teils auf die kontinuierliche Bahn (2) aus Artikelhalbfertigteilen in vorbestimmten Intervallen in Längsrichtung der kontinuierlichen Bahn während des Laufs der Bahn aufgebracht werden, während die elastischen Teile in dem quer zur kontinuierlichen Bahn gedehnten Zustand gehalten werden, und wobei die elastischen Teile an der kontinuierlichen Bahn befestigt werden, und
- Mittel (12'-14') zum wiederholten Trennen der kontinuierlichen Bahn (2) entlang einer Trennlinie (26), die sich quer zur Bahn erstreckt, welche Teile sich jeweils auf den unmittelbar benachbarten Enden aufeinanderfolgender individueller Artikel befinden dadurch **gekennzeichnet**, daß
daß die Zuführungs-, die Dehn- und die Transfermittel folgende Merkmale aufweisen:
- erste und zweite Walzenpaare (16,17,19,20),
- erste und zweite Paare Endlosgurte (18a,18b,21a,21b), die auf den jeweiligen Walzenpaaren zum Zwecke des Förderantriebs herumgeführt sind,
- die Walzen jedes Paars sind in Laufrichtung voneinander beabstandet und jeweils gegenüber der Schneide-Station (12-14) und den Transfermitteln (20) angeordnet,
- Mittel (16a,16b,17a,17b,19a,19b,20a,20b) auf den Walzen, um die Gurte jedes Paars in divergierendem Verhältnis im Abstand zu halten, wenn sie sich in Laufrichtung von der Schneidestation zu den Transfermitteln erstrecken, wobei die Gurte des ersten Paars mit dem gleichen Winkel divergieren wie die Gurte des zweiten Paars,
- die ersten und zweiten Walzenpaare sind jeweils in einander gegenläufigem Drehsinn angetrieben und so angeordnet, daß die jeweiligen angetriebenen Trume der Gurte der ersten Gurtpaare, die sich in Zuführungsrichtung erstrecken, mit den angetriebenen Trumen der jeweiligen Gurte des zweiten Gurtpaares fluchten und ihnen gegenüber liegen, so daß die jeweiligen Gurte des ersten Paars mit den jeweiligen Gurten des zweiten Paars zusammenarbeiten, um zwischen sich die Enden der elastischen Teile (7) zu ergreifen um die aufeinanderfolgend ein elastischen Teile von der Schneidestation zur Transferstation zu überführen, währenddessen die elastischen Teile in Querrichtung gedehnt werden und
- eine Walze (20) des zweiten Paars (19,20) ist dem Vorrat der Bahn (2) benachbart und weist Mittel (22a,22b) zum Aufnehmen und lösbaren Halten der elastischen Teile auf, die von den Gurten freigegeben sind, um die beschichteten Oberflächen aufeinanderfolgend gegen die Bahn zu pressen und dabei die elastischen Teile auf die Bahn zu übertragen.

2. Vorrichtung nach Anspruch 1, dadurch **gekennzeichnet,** daß die Mittel zu Halten der Bahn (2) eine rotierende Trommel (25) aufweisen, um die sich die Bahn herumerstreckt.

3. Vorrichtung nach Anspruch 1 oder 2, dadurch **gekennzeichnet,** daß die Mittel (12'-14') zum Abtrennen der kontinuierlichen Bahn (2) so angeordnet sind, daß die Trennlinie (26) durch einen vorbestimmten Bereich der jeweiligen aufeinanderfolgenden elastischen Teile (7) hindurchführt und dabei jedes elastische Teil in Laufrichtung der Bahn in zwei Teilehälften trennt, die an den einander zugewandten Enden aufeinanderfolgender einzelner Artikel angeordnet sind.

4. Verfahren zum Befestigen eines elastischen Teils an einem tragbaren Artikel, unter Verwendung der Vorrichtung nach einem der Ansprüche 1 bis 3, mit den folgenden Verfahrensschritten:
a) Zuführen einer kontinuierlichen elastischen Lage (77) vorbestimmter Breite von einem Vorrat zu einer Schneidestation (12-14), wahrend ein Beschichten der kontinuierlichen elastischen Lage auf einer Oberfläche mit einem Klebmittel (A) durchgeführt wird, wobei die einander gegenüberliegenden Längsränder freigelassen werden,
b) wiederholtes Schneiden an der Schneidestation der kontinuierlichen elastischen Lage entlang einer Trennlinie (26), die sich in Querrichtung erstreckt, um aufeinanderfolgende individuelle elastische Teile (7) vorbestimmter Länge auszubilden,
c) Zuführen der auf diese Weise gebildeten individuellen elastischen Teile aus der Schneidestation zu einer Transferstation (20), während jedes elastische Teil zu einem in Querrichtung gedehnten Zustand gestreckt wird,
d) Aufbringen der beschichteten Seite jedes einzelnen elastischen Teils auf eine kontinuierliche Bahn (2) aus Artikelhalbfertigteilen in in Längsrichtung der Bahn vorbestimmten Abständen, während das elastische Teil in dem quer zur kontinuierlichen Bahn gedehnten Zustand gehalten wird, und
- wiederholtes Abschneiden der kontinuierlichen Bahn entlang einer Trennlinie (26), die sich durch einen vorbestimmten Abschnitt der jeweiligen aufeinanderfolgenden elastischen Teile erstreckt quer zur Bahn, wobei jedes elastische Teil in Laufrichtung in zwei Teile geteilt, wobei die getrennten Einzelteile jeweils auf den sich gegenüberliegenden Enden zweier aufeinanderfolgender einzelner Artikel angeordnet sind, dadurch **gekennzeichnet,** daß die elastischen Teile (7) zwischen in Laufrichtung divergierend geführten Gurtpaaren (18a,18b,21a,21b) auf eine vorbestimmte Länge gedehnt und an eine Andruckwalze (20) der Transportstation mittels Unterdruck auf einer Saugvorrichtung (22a,22b) zum Aufkleben auf ein zugeordnetes Halbertigteil gehalten werden.

## Claims

1. A device for fixing an elastic part to an article of apparel, with the following features:
- means (9,10, 15) for feeding a continuous elastic layer (77) of predetermined width from a supply to a cutting station (12, 13, 14)
- means (11) for continuous application of an adhesive coating A to a surface of the elastic layer with the exception of marginal regions extending in the longitudinal direction,
- feed means (24, T₁, T₂) which run from the cutting station to transfer means (20), in order to transport the individual, successively formed elastic pieces (7) cut off transversely at the cutting station from the cutting station to the transfer means with the elastic pieces spaced from one another by a predetermined distance in the running direction,
- means (T₁, T₂, 16, 17, 19, 20, 23) for stretching the elastic pieces into a stretched state in the transverse direction,
- means (25) for feeding a continuous band (2) of semi-finished articles to the transfer means, which are so arranged that the coated surface of each elastic piece is applied to the continuous band (2) of semi-finished articles at predetermined intervals in the longitudinal direction of the continuous band, during the movement of the band, while the elastic pieces are maintained in the stretched state transverse to the continuous band, and whereby the elastic pieces are fixed to the continuous band, and
- means (12'-14') for repeated separation of the continuous band (2) along a line of separation (26) which extends transverse to the band, which position pieces on each of the directly adjoining ends of individual articles following on one another, characterized in that the feed means, the stretching means and the transfer means have the following features:
- first and second roller pairs (16, 17, 19, 20),
- first and second pairs of endless belts (18a, 18b, 21a, 21b) which are led round the respective roller pairs in order to provide conveyor drive,
- the rollers of each pair are spaced apart in the longitudinal direction and arranged opposite the cutting station (12-14) and the transfer means (20),
- means (16a, 16b, 17a, 17b, 19a, 19b, 20a, 20b) on the rollers for maintaining the belts of each pair spaced in a divergent relationship, when they extend in the running direction from the cutting station to the transfer means, where the belts of the first pair diverge at the same angle as the belts of the second pair,
- the first and second roller pairs are driven in opposite senses of rotation to one another and are so arranged that the respective drive runs of the belts of the first belt pair which run in the feed direction are aligned with the drive runs of the respective belts of the second belt pair and lie opposite the same, so that the respective belts of the first pair cooperate with the respective belts of the second pair, in order to grip therebetween the ends of the elastic pieces (7) in order to transfer the sequentially following elastic pieces from the cutting station to the transfer station while the elastic pieces are stretched in the transverse direction, and
- a roller (20) of the second pair (19, 20) is adjacent the supply of the band (2) and comprises means (22a, 22b) for receiving and releasably retaining the elastic pieces which are released from the belts, in order to press the coated surfaces one after the other against the band and thus transfer the elastic pieces to the band.

2. A device according to claim 1, characterized in that the means for holding the band (2) comprise a rotating drum (25), about which the band extends.

3. A device according to claim 1 or 2, characterized in that the means (12'-14') for separating the continuous band (2) are so arranged that the line of separation (26) passes through a predetermined region of the successive elastic pieces (7) so that each piece is divided in the running direction into two half pieces, which are arranged on the facing ends of successive individual articles.

4. A method of fixing an elastic piece to an article of wear, using a device according to any one of claims 1 to 3, with the following method steps:
a) feeding a continuous elastic layer (77) of predetermined width from a supply to a cutting station (12, 13, 14) while coating of the continuous elastic layer with an adhesive (A) on one surface is performed, with the opposite longitudinal margins being left free,
b) repeated cutting at the cutting station of the continuous elastic layer along a line of separation (26) which extends in the transverse direction in order to form successive individual elastic pieces (7) of predetermined length,
c) feeding individual elastic pieces formed in this manner from the cutting station to a transfer station (20) while each elastic piece is stretched to a stretched state in the transverse direction,
d) applying the coated side of each individual elastic piece to a continuous band of semi-finished articles at predetermined intervals along the longitudinal direction of the band, while the elastic piece is maintained in the stretched state transverse to the continuous band, and
- repeated cutting off of the continuous band along a line of separation (26) which extends transverse to the band through a predetermined section of the respective successive elastic pieces, whereby each elastic piece is divided in the ruming direction into two parts, where the separated individual parts are arranged on the mutually opposed ends of two successive individual articles, characterized in that the elastic pieces (7) are stretched to a predetermined length between pairs of belts (18a, 18b, 21a, 21b) diverging in the running direction and are held on a pressure roller (20) of the transport station by means of vacuum on a suction device (22a, 22b) for the sticking on to an associated semi-finished part.

## Revendications

1. Dispositif pour fixer un élément élastique sur un article portable, présentant les caractéristiques suivantes :
- des moyens (9, 10, 15) pour amener une couche élastique continue (77) de largeur prédéterminée, d'un poste de stockage à un poste de coupe (12, 13, 14),
- des moyens (11) pour appliquer en continu une couche adhésive (A) sur une surface de la couche élastique, à l'exception des zones marginales qui s'étendent dans le sens longitudinal,
- des moyens (24, T₁, T₂), qui s'étendent du poste de coupe jusqu'à des moyens de transfert (20), en vue de transporter depuis le poste de coupe jusqu'aux moyens de transfert, les éléments élastiques (7) individuels, produits successivement et découpés transversalement à la bande dans le poste de coupe, les éléments élastiques étant tenus équidistants dans le sens de l'avancement, sur une distance prédéterminée,
- des moyens (T₁, T₂, 16, 17, 19, 20, 23) pour étirer les éléments élastiques dans le sens transversal,
- des moyens (25) pour amener une bande continue (2) d'éléments semi-finis de l'article jusqu'aux moyens de transfert, moyens disposés de manière à ce que la surface enduite de chaque élément élastique soit appliquée sur la bande continue (2) constituée d'éléments semi-finis de l'article, à des intervalles prédéterminés dans le sens longitudinal de la bande continue, au cours de la progression de ladite bande, alors que les éléments élastiques sont maintenus à l'état étiré transversalement à la bande continue, les éléments élastiques étant attachés à la bande continue, et,
- des moyens (12'-14') pour séparer la bande continue (2) de manière répétitive, en suivant une ligne de séparation (26) qui s'étend transversalement à la bande, éléments situés respectivement sur les extrémités immédiatement voisines d'articles individuels successifs, ce dispositif étant caractérisé en ce que les moyens pour amener, étirer et transférer présentent les caractéristiques suivantes :
- des premières et secondes paires de rouleaux (16, 17, 19, 20),
- des premières et secondes paires de courroies continues (18a, 18b, 21a, 21b), guidées autour des paires de rouleaux respectives à des fins d'entraînement,
- les rouleaux de chaque paire sont écartés l'un de l'autre dans le sens de l'avancement et disposés respectivement face au poste de coupe (12-14) et face aux moyens de transfert (20),
- des moyens (16a, 16b, 17a, 17b, 19a, 19b, 20a, 20b) prévus sur les rouleaux, pour maintenir les courroies de chaque paire écartées dans un rapport divergent, lorsqu'elles s'étendent du poste de coupe aux moyens de transfert dans le sens de l'avancement, les courroies de la première paire divergeant selon le même angle que les courroies de la seconde paire,
- les première et seconde paires de rouleaux sont entraînées dans un sens de rotation antagoniste et disposées de manière à ce que les brins tendus des courroies de la première paire de courroies, qui s'étendent dans le sens de l'amenée, s'alignent sur les brins tendus des courroies de la seconde paire de courroies et leur font face, de sorte que les courroies de la première paire travaillent de concert avec les courroies respectives de la seconde paire, afin de saisir entre elles les extrémités des éléments élastiques (7) et de convoyer les éléments élastiques successifs jusqu'au poste de transfert, en étirant les éléments élastiques dans le sens transversal, et
- un rouleau (20) de la seconde paire (19, 20) est voisin de la réserve de bande (2) et présente des moyens (22a, 22b) pour soulever et retenir, d'une manière qui permet de les relâcher, les éléments élastiques qui ont été libérés par les courroies, afin de presser successivement les surfaces enduites contre la bande et ainsi transférer les éléments élastiques sur la bande.

2. Dispositif suivant la revendication 1, caractérisé en ce que les moyens pour retenir la bande (2) comprennent un tambour rotatif (25) autour duquel s'enroule la bande.

3. Dispositif suivant l'une des revendications 1 ou 2, caractérisé en ce que les moyens (12'-14') pour découper la bande continue (2) sont disposés de telle manière que la ligne de séparation (26) traverse une zone prédéterminée des éléments élastiques successifs (7), et partage chaque élément élastique dans le sens de l'avancement de la bande en deux moitiés, qui sont appliquées sur les extrémités d'articles individuels successifs tournées l'une vers l'autre.

4. Procédé pour fixer un élément élastique sur un article portable, avec utilisation du dispositif conforme à l'une des revendications 1 à 3, consistant en la séquence d'opérations suivantes :
a) amenée d'une couche élastique continue (77) de largeur prédéterminée d'un poste de stockage, jusqu'à un poste de coupe (12), tandis qu'un adhésif (A) est appliqué sur une surface de ladite couche élastique continue, les bords longitudinaux mutuellement opposés de cette dernière étant laissés libres,
b) découpe répétitive de la couche élastique continue dans le poste de coupe, en suivant une ligne de séparation transversale (26), en vue de former des éléments élastiques individuels (7) successifs de longueur prédéterminée,
c) amenée des éléments élastiques individuels ainsi produits, du poste de coupe à un poste de transfert (20), en étirant chaque élément élastique de manière à l'allonger dans le sens transversal,
d) application du côté enduit de chaque élément élastique sur une bande continue (2) d'éléments semi-finis de l'article, à des intervalles prédéterminés dans le sens longitudinal de la bande, en maintenant l'élément élastique à l'état étiré transversalement à la bande continu, et
- découpe répétitive de la bande continue en suivant une ligne de séparation (26) qui s'étend transversalement à la bande à travers une portion ou section prédéterminée des éléments élastiques successifs, chaque élément élastique étant partagé dans le sens d'avancement en deux parties, les parties individuelles séparées étant respectivement appliquées sur les extrémités opposées de deux articles individuels successifs, caractérisé en ce que les éléments élastiques (7) sont étirés entre des paires de courroies (18a, 18b ; 21a, 21b) guidées dans un rapport divergent dans le sens de l'avancement, et maintenus appliqués sur un rouleau de pression (20) du poste de transfert en mettant un dispositif de succion (22a, 22b) sous vide, en vue de les coller à un élément semi-fini correspondant.
